(19) Europäisches Patentamt
European Patent Office
Office européen des brevets

(11) **EP 3 639 891 A1**

(12) **EUROPEAN PATENT APPLICATION**
published in accordance with Art. 153(4) EPC

(43) Date of publication:
**22.04.2020 Bulletin 2020/17**

(51) Int Cl.:
***A61N 1/44*** *(2006.01)*     ***A61C 19/06*** *(2006.01)*

(21) Application number: **18816666.4**

(22) Date of filing: **15.06.2018**

(86) International application number:
**PCT/JP2018/022868**

(87) International publication number:
**WO 2018/230696 (20.12.2018 Gazette 2018/51)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **16.06.2017   JP 2017119153**

(71) Applicant: **Sekisui Chemical Co., Ltd.
Osaka 530-8565 (JP)**

(72) Inventors:
• **NAGAHARA, Yu
Kyoto-shi
Kyoto 601-8105 (JP)**

• **OSHITA, Takaya
Kyoto-shi
Kyoto 601-8105 (JP)**
• **TAKIKAWA, Yoshishige
Kyoto-shi
Kyoto 601-8105 (JP)**
• **UEHARA, Tsuyoshi
Kyoto-shi
Kyoto 601-8105 (JP)**

(74) Representative: **Ter Meer Steinmeister & Partner
Patentanwälte mbB
Nymphenburger Straße 4
80335 München (DE)**

(54) **ACTIVE GAS EXPOSURE DEVICE AND METHOD FOR TREATING NON-HUMAN ANIMALS**

(57)     A reactive gas application apparatus including an instrument (10) which includes: a plasma generating unit (12); and an outlet (1a) for blowing out a reactive gas activated by plasma, the plasma generating unit (12) including: a tubular dielectric (3) to which a plasma generation gas is introduced; an inner electrode (4) provided inside the tubular dielectric (3), and extending in a tube axis direction (O1) of the tubular dielectric (3); and an outer electrode (5) provided outside the tubular dielectric (3) and faces the inner electrode (4) through the tubular dielectric, wherein the inner electrode (4) has a shaft portion extending in the tube axis direction and a spiral ridge formed on an outer peripheral surface of the shaft portion.

FIG. 2

**Description**

TECHNICAL FIELD

The present invention relates to a reactive gas application apparatus, and a method of treating animals excluding humans.

Priority is claimed on Japanese Patent Application No. 2017-119153, filed June 16, 2017, the contents of which are incorporated herein by reference.

BACKGROUND ART

Conventionally, apparatuses for medical use such as dental treatment are known, which apply plasma to an affected part of a patient for healing wounds and the like.

For example, Patent Document 1 discloses a dental diagnosis apparatus in which a plasma jet application means is mounted on an instrument for performing dental treatment so as to enable plasma jet application to an affected part.

According to the invention described in Patent Document 1, the generated plasma is directly applied to the affected part in an attempt to heal the wound and the like.

Prior Art References

Patent Document

Patent Document 1: Japanese Patent Granted Publication No. 5441066

DISCLOSURE OF INVENTION

Problems to be Solved by the Invention

As plasma application apparatuses, there are a plasma jet application apparatus and a reactive gas application apparatus.

The plasma jet application apparatus generates plasma and directly applies the generated plasma together with reactive species to a target object, in which the reactive species are generated by reaction within the plasma or reaction of the plasma with ambient gas. Examples of the reactive species include reactive oxygen species such as hydroxyl radical, singlet oxygen, ozone, hydrogen peroxide and superoxide anion radical, and reactive nitrogen species such as nitric oxide, nitrogen dioxide, peroxynitrite and dinitrogen trioxide.

The reactive gas application apparatus generates plasma and applies the generated plasma together with reactive species to a target object, in which the reactive species are generated by reaction within the plasma or reaction of the plasma with ambient gas.

In addition, in order to suppress the temperature rise of a target surface and to enhance the effect of wound healing and the like, it is necessary to stably generate plasma with a relatively low voltage.

Accordingly, it is an object of the present invention to provide a reactive gas application apparatus capable of further enhancing the effect of plasma.

Means to Solve the Problems

The embodiments of the present invention are as follows.

[1] A reactive gas application apparatus including an instrument, the instrument including:

> a plasma generating unit, and
> an outlet for blowing out a reactive gas activated by plasma,
> the plasma generating unit including:

>> a tubular dielectric to which a plasma generating gas is introduced;
>> an inner electrode provided inside the tubular dielectric and extending in a tube axis direction of the tubular dielectric; and
>> an outer electrode provided outside the tubular dielectric and facing the inner electrode through the tubular dielectric,

wherein the inner electrode has a shaft portion extending in the tube axis direction and a helical ridge formed on an outer peripheral surface of the shaft portion.

[2] The reactive gas application apparatus according to [1], wherein a crest of the helical ridge has an acute angle.
[3] The reactive gas application apparatus according to [1] or [2], wherein an interval between the crests of neighboring turns of the helical ridge is 0.2 to 3.0 mm as viewed in the tube axis direction.
[4] The reactive gas application apparatus according to any one of [1] to [3], wherein a height of the helical ridge is 0.1 to 3.0 mm.
[5] The reactive gas application apparatus according to any one of [1] to [4], wherein a length of a region where the inner electrode faces the outer electrode is 1 to 50 mm.
[6] The reactive gas application apparatus according to any one of [1] to [5], wherein the plasma generating gas contains nitrogen.
[7] The reactive gas application apparatus according to [6], wherein the plasma generating gas has a nitrogen content of 80 to 100 % by volume with respect to a total volume of the plasma generating gas.
[8] The reactive gas application device according to any one of [1] to [7], which is a medical therapeutic apparatus.
[9] The reactive gas application apparatus according to any one of [1] to [7], which is a therapeutic apparatus for treating animals excluding humans.
[10] A method of treating animals excluding humans, comprising providing the reactive gas application apparatus according to any one of [1] to [7], and applying the reactive gas to a tissue of an animal excluding humans.

In the present specification, the reactive gas refers to a gas having high chemical activity and including any of reactive species such as radicals, excited atoms, excited molecules, ions, and the like.

Effect of the Invention

According to the reactive gas application apparatus of the present invention, the effect of plasma can be further enhanced.

BRIEF DESCRIPTION OF THE DRAWINGS

FIG. 1 is a schematic view of a reactive gas application apparatus according to an embodiment of the present invention.
FIG. 2 is a partial cross-sectional view of an instrument of a reactive gas application apparatus according to an embodiment of the present invention.
FIG. 3 is a cross-sectional view of the instrument of FIG. 2 as viewed from the arrow direction of the x-x line of FIG. 2.
FIG. 4 is a side view of an inner electrode according to another embodiment of the present invention.

DESCRIPTION OF THE EMBODIMENTS

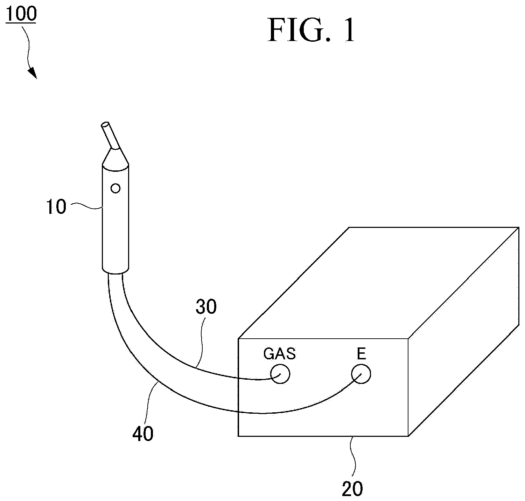

The reactive gas application apparatus of the present invention includes an instrument including a plasma generating unit and an outlet for blowing out a reactive gas activated by plasma.
Hereinbelow, an example of the reactive gas application apparatus of the present invention will be described.
The reactive gas application apparatus 100 shown in FIG. 1 includes an instrument 10, a power supply unit 20, a gas conduit 30, and an electrical wiring 40.
The gas conduit 30 is connected to the instrument 10 and the power supply unit 20. The electrical wiring 40 is connected to the instrument 10 and the power supply unit 20.
In the present embodiment, the gas conduit 30 and the electric wiring 40 are provided independently from each other, but the gas conduit 30 and the electric wiring 40 may be bundled together.
The power supply unit 20 is connected to a plasma generating gas supply source (not shown).

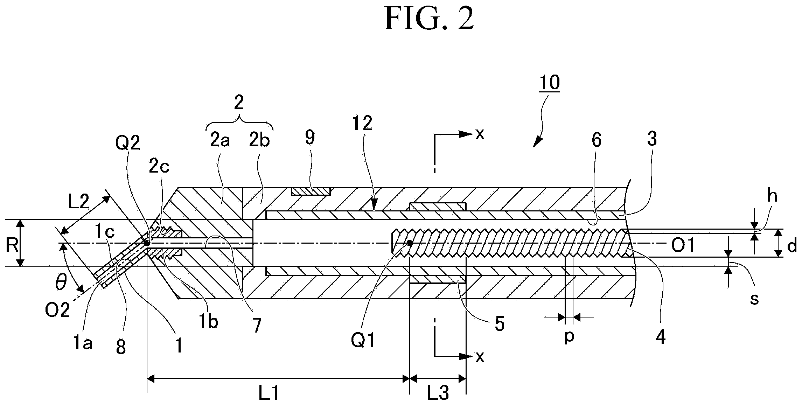

FIG. 2 is a cross-sectional (longitudinal section) view showing a plane along the axis of the instrument 10. The instrument 10 is a device that blows out reactive species.
As shown in FIG. 2, the instrument 10 includes an elongated cowling 2, a nozzle 1 provided at the tip of the cowling 2, and a plasma generating unit 12 provided in the cowling 2.
The cowling 2 includes a cylindrical body 2b and a head 2a provided at the tip of the body 2b. The body 2b is not limited to that of a cylindrical shape, and may be of a polygonal tube shape such as a square tube shape, a hexagonal tube shape, an octagonal tube shape or the like.

The head 2a gradually narrows toward the tip thereof. That is, the head 2a in the present embodiment has a conical shape. The head 2a is not limited to that of a conical shape, and may be of a polygonal cone shape such as a quadrangular pyramid shape, a hexagonal pyramid shape, an octagonal pyramid shape or the like.

A fitting hole 2c into which the nozzle 1 is inserted is formed at the tip of the head 2a. The nozzle 1 is detachably attached to the head 2a. Symbol O1 denotes the tube axis of the body 2b. A first reactive gas flow path 7 extending in the tube axis O1 direction is formed inside the head 2a.

A switch 9 is provided on the outer peripheral surface of the body 2b.

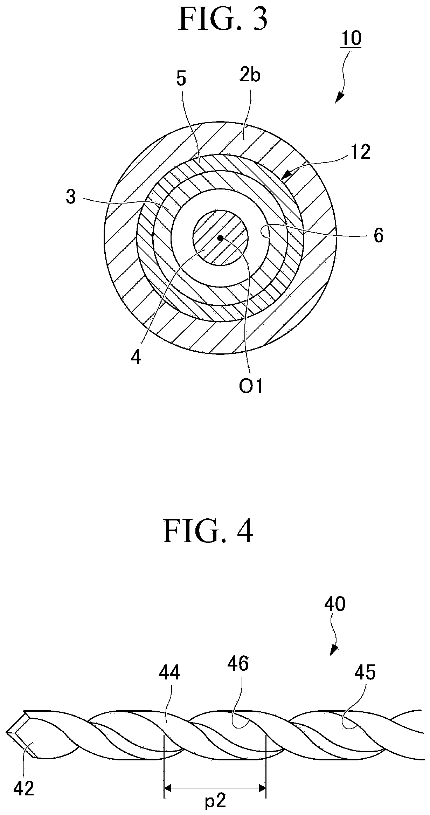

As shown in FIGs. 2 and 3, the plasma generating unit 12 includes a tubular dielectric 3, an inner electrode 4, and an outer electrode 5.

The tubular dielectric 3 is a cylindrical member extending in the tube axis O1 direction. A gas flow path 6 extending in the tube axis O1 direction is formed inside the tubular dielectric 3. The gas flow path 6 communicates with a first reactive gas flow path 7. The tube axis O1 coincides with the tube axis of the tubular dielectric 3.

In the tubular dielectric 3, an inner electrode 4 is provided. The inner electrode 4 is a substantially columnar member extending in the tube axis O1 direction. The inner electrode 4 is spaced apart from the inner surface of the tubular dielectric 3.

On the outer peripheral surface of the tubular dielectric 3, an outer electrode 5 extending along the inner electrode 4 is provided. The outer electrode 5 surrounds the outer periphery of the tubular dielectric 3. That is, the outer electrode 5 is provided outside the tubular dielectric 3.

The shape of the outer electrode 5 in the present embodiment is cylindrical. The shape of the outer electrode 5 is not limited to a cylindrical shape, and may be a rod shape or a plate shape. When the outer electrode 5 is in the shape of a rod or a plate, the number of the outer electrode 5 may be one or two or more.

As shown in FIG. 3, the tubular dielectric 3, the inner electrode 4, and the outer electrode 5 are positioned concentrically around the tube axis O1.

In the present embodiment, the outer peripheral surface of the inner electrode 4 and the inner peripheral surface of the outer electrode 5 face each other through the tubular dielectric 3.

The nozzle 1 includes a base 1b fitted in the fitting hole 2c, and an outlet tube 1c. The base 1b and the outlet tube 1c are integrally formed. A second reactive gas flow path 8 is formed inside the nozzle 1, and an outlet 1a is formed at the tip of the nozzle 1. The second reactive gas flow path 8 and the first reactive gas flow path 7 communicate with each other.

The material of the body 2b is not particularly limited, but is preferably an insulating material. Examples of the insulating material include thermoplastic resins such as polyethylene, polypropylene, polyvinyl chloride and polystyrene; and thermosetting resins such as a phenol resin, a melamine resin, a urea resin, an epoxy resin and an unsaturated polyester resin.

The size of the body 2b is not particularly limited, and is such a size that allows the body 2b to be easily grasped with fingers.

The material of the head 2a is not particularly limited, and may or may not be an insulating material. The material of the head 2a is preferably a material excellent in abrasion resistance and corrosion resistance. As such a material excellent in abrasion resistance and corrosion resistance, a metal such as stainless steel can be mentioned. The materials of the head 2a and the body 2b may be the same or different.

The size of the head 2a is set in consideration of the use of the reactive gas application device 100 and the like. For example, when the reactive gas application apparatus 100 is an apparatus for an intraoral treatment, the size of the head 2a is set to be large enough so that the apparatus 100 can be inserted into an oral cavity.

As a material of the tubular dielectric 3, a dielectric material used for a known plasma generator can be employed. Examples of the material of the tubular dielectric 3 include glass, ceramics, synthetic resins, and the like. The dielectric constant of the tubular dielectric 3 is preferably as low as possible.

The inner diameter R of the tubular dielectric 3 is appropriately set in consideration of the outer diameter d of the inner electrode 4. The inner diameter R is set such that a distance s (described later) falls within a predetermined range.

The inner electrode 4 includes a shaft portion extending in the tube axis O1 direction and a screw thread formed on the outer peripheral surface of the shaft portion. That is, the inner electrode 4 includes a shaft portion and a helical ridge formed on the outer peripheral surface of the shaft portion. The shaft portion may be solid or hollow. Of these, a solid shaft portion is more preferable. The solid shaft portion allows easy processing and improves mechanical durability. The screw thread of the inner electrode 4 is a helical screw thread that circulates in the circumferential direction of the shaft portion. The shape of the inner electrode 4 is the same as that of a screw or a bolt.

The inner electrode 4 may be what is referred to as a parallel screw or a tapered screw. The parallel screw is a screw having the same thickness from its base to its tip. The tapered screw is a screw which gradually narrows from its base toward its tip.

The direction of the screw thread in the inner electrode 4 is not particularly limited. The screw shape of the inner electrode 4 may be that of what is referred to as a right-handed screw (a screw that advances in the direction pointed by its tip when rotated to the right) or that of what is referred to as a left-handed screw.

The shape of the screw thread of the inner electrode 4 is not particularly limited, and the screw thread may be triangular, square, or trapezoidal. The triangular thread is a thread having a triangular cross section as viewed in the longitudinal cross section of a screw or a bolt. The square thread is a thread having a square cross section as viewed in the longitudinal cross section of a screw or a bolt. The trapezoidal thread is one type of square threads and is a thread with its cross section gradually narrowing toward its tip.

Plasma is more easily generated when the thread crest of the inner electrode 4 is sharp. For this reason, the screw thread is preferably triangular, trapezoidal or the like, more preferably triangular. Further, the thread crest preferably has an acute angle (less than 90 °). The angle of the thread crest is more preferably from 30 to 90 °, still more preferably from 45 to 70 °. Plasma can be generated more efficiently when the angle of thread crest is within the above range. The angle of thread crest is an angle of the tip of screw thread as viewed with respect to its cross section orthogonal to the direction in which the screw thread extends.

With respect to the screw thread 4 of the inner electrode 4, the inner electrode 4 may be in the form of a single-threaded screw having one thread per one pitch which is a distance between the crest of an arbitrarily chosen thread and the crest of the neighboring thread (namely, a screw with a single screw groove) or a multi-threaded screw having two or more threads per one pitch (namely, a screw with two or more adjacent screw grooves).

The outer diameter d of the inner electrode 4 is appropriately set in consideration of the application of the reactive gas application apparatus 100 (that is, the size of the instrument 10) and the like. When the reactive gas application apparatus 100 is an apparatus for an intraoral treatment, the outer diameter d is preferably 0.5 to 20 mm, more preferably 1 to 10 mm. When the outer diameter d is not less than the above lower limit value, the inner electrode can be easily manufactured. Further, the outer diameter d of not less than the above lower limit value increases the surface area of the inner electrode 4, whereby plasma can be generated more efficiently, and healing and the like can be further promoted. When the outer diameter d is not more than the above upper limit value, plasma can be generated more efficiently and the healing and the like can be further promoted without excessively increasing the size of the instrument 10.

The height h of the screw thread (ridge) of the inner electrode 4 can be appropriately set in consideration of the outer diameter d of the inner electrode 4. The height h of the screw thread is preferably, for example, 0.1 to 3.0 mm. Plasma can be generated more efficiently when the height h is not less than the above lower limit value. When the height h is not more than the above upper limit value, the strength of the inner electrode 4 can be increased. The height h of the screw thread is a height in a direction perpendicular to the tube axis O1 and is a length from the bottom of the valley formed between the turns of screw thread to the crest of the screw thread.

The distance between the crests of the neighboring screw thread turns in the tube axis O1 direction is the thread pitch p of the screw.

The thread pitch p of the inner electrode 4 can be appropriately set in consideration of the length and outer diameter d of the inner electrode 4, and the like. The thread pitch p is preferably, for example, 0.2 to 3.0 mm, more preferably 0.2 to 2.5 mm, and still more preferably 0.2 to 2.0 mm. When the pitch p is not less than the above lower limit value, plasma can be generated even more easily. When the pitch p is not more than the above upper limit value, plasma generation sites can be increased to generate plasma more efficiently. When the inner electrode 4 is in the form of a multi-threaded screw, the pitch p does not mean an interval between the crests of the same thread but an interval between the crest of a predetermined thread and the crest of the other thread adjacent thereto (i.e., a distance taken between the crests of neighboring threads disregarding the crest of adjacent turn of the same tread).

The material constituting the inner electrode 4 is not particularly limited as long as the material is electrically conductive, and metals used for electrodes of known plasma generating apparatuses can be used. Examples of the material of the inner electrode 4 include metals such as stainless steel, copper and tungsten, carbon, and the like.

The inner electrode 4 preferably has the same specification as any of the metric screw threads complying with JIS B 0205: 2001 (M2, M2.2, M2.5, M3, M3.5, etc.), the metric trapezoidal screw threads complying with JIS B 2016: 1987 (Tr8 × 1.5, Tr9 × 2, Tr9 × 1.5, etc.), the unified coarse screw threads complying with JIS B 0206: 1973 (No. 1 - 64 UNC, No. 2 - 56 UNC, No. 3 - 48 UNC, etc.), and the like. The inner electrode 4 having the same specification as those standardized products is advantageous in terms of cost.

The distance s between the outer surface of the inner electrode 4 (the crest of the screw thread) and the inner surface of the tubular dielectric 3 is preferably 0.05 to 5 mm, more preferably 0.1 to 1 mm. When the distance s is not less than the above lower limit value, a desired amount of gas is allowed to flow easily. When the distance s is not more than the above upper limit value, plasma can be generated more efficiently and the temperature of the reactive gas can be further lowered.

The length L3 of the region where the inner electrode 4 faces the outer electrode 5 is preferably 1 to 50 mm, more preferably 3 to 40 mm, and even more preferably 5 to 30 mm. When the length L3 is not less than the above lower limit value, plasma generation sites can be increased to generate plasma more efficiently. When the length L3 is not more than the above upper limit value, the temperature rise of the reactive gas can be more favorably suppressed. In the present embodiment, the length L3 is equal to the length of the outer electrode 5.

The outer electrode 5 may be divided into two or more sections in the direction of tube axis O1. When the outer electrode 5 is divided in the direction of tube axis O1, the length L3 is a length from the rear end to the tip end of the combination of the two outer electrodes and includes a distance (gap length) between the two outer electrodes.

The shape of the inner electrode is not limited to that of a screw or a bolt. The inner electrode may be the inner electrode 40 as shown in FIG. 4. The inner electrode 40 includes a shaft portion 42 and a spiral ridge 44. The inner electrode 40 is provided with a ridge 44 having a width larger than that of the inner electrode 4. The inner electrode 40 has a drill-like shape. The pitch p2 in the inner electrode 40 is long, for example, as compared to the pitch p in the inner electrode 4. The pitch p2 is, for example, 3 to 20 mm. The ridge 44 has a flat crest. Therefore, the pitch p2 coincides with the interval between the edges 46 of the ridge 44. The edge 46 is an edge of ridge 44, which is located on the forward end side of the inner electrode 40.

The inner electrode preferably has a screw-like or bolt-like shape. When the inner electrode has a screw-like or bolt-like shape, the pitch is short, so that more plasma generation sites can be formed in the region where the inner electrode and the outer electrode face each other. For this reason, the inner electrode having a screw-like or bolt-like shape can generate plasma more efficiently.

The material constituting the outer electrode 5 is not particularly limited as long as the material is electrically conductive, and metals used for electrodes of known plasma generating apparatuses can be used. Examples of the material of the outer electrode 5 include metals such as stainless steel, copper and tungsten, carbon, and the like.

The material of the nozzle 1 is not particularly limited, and may or may not be an insulating material. The material of the nozzle 1 is preferably a material excellent in abrasion resistance and corrosion resistance. As such a material excellent in abrasion resistance and corrosion resistance, a metal such as stainless steel can be mentioned.

The length (that is, the distance L2) of the flow path in the outlet tube 1c can be appropriately set in consideration of the use of the reactive gas application apparatus 100 or the like.

The opening diameter of the outlet 1a is preferably, for example, 0.5 to 5 mm. When the opening diameter is not less than the above lower limit value, the pressure loss of the reactive gas can be suppressed. When the opening diameter is not more than the above upper limit value, the flow rate of the blown out reactive gas can be increased to further promote healing and the like.

The outlet tube 1c is bent with respect to the tube axis O1.
The angle θ formed between the tube axis O2 of the outlet tube 1c and the tube axis O1 can be set in consideration of the use of the reactive gas application apparatus 100 and the like. The angle θ is preferably, for example, 0 to 90 °, more preferably 10 to 60 °.

The sum of the distance L1 from the tip center point Q1 of the outer electrode 5 to the tip end Q2 of the head 2a and the distance L2 from the tip end Q2 to the outlet 1a (that is, a distance from the inner electrode 4 to the outlet 1a) is appropriately set in consideration of the size of the reactive gas application apparatus 100, the temperature of the reactive gas on a surface to which the reactive gas is applied (target surface), and the like. When the sum of the distance of L1 and the distance L2 is large, the reactive gas temperature at the target surface can be further lowered. When the sum of the distance of L1 and the distance L2 is small, the radical concentration of the reactive gas can be further increased, and the effects of cleaning, activation, healing, etc. on the target surface can be further enhanced. The tip end Q2 is an intersection point between the tube axis O1 and the tube axis O2.

The temperature of the reactive gas at a target surface can be measured by a thermocouple.

The power supply unit 20 is a device that transmits electric power to the instrument 10. The power supply unit 20 in the present embodiment is provided with a pump that sends a plasma generating gas to the instrument 10 via the gas conduit 30. The power supply unit 20 can control the voltage to be applied between the outer electrode 5 and the inner electrode 4, and the frequency thereof.

The power supply unit 20 may not have a pump. In such case, a pump may be provided independently of the power supply unit 20. Alternatively, the plasma generating gas may also be supplied to the instrument 10 by pressure at the plasma generating gas supply source.

The gas conduit 30 is a path for supplying the plasma generating gas from the power supply unit 20 to the instrument 10. The gas conduit 30 is connected to the rear end of the tubular dielectric 3 of the instrument 10. The material of the gas conduit 30 is not particularly limited, and a material used for known gas pipes can be applied. Concerning a material of the gas conduit 30, for example, a resin pipe, a rubber tube and the like can be used, and a material having flexibility is preferable.

The electrical wiring 40 is a wiring for supplying electricity from the power supply unit 20 to the instrument 10. The electric wiring 40 is connected to the inner electrode 4, the outer electrode 5 and the switch 9 of the instrument 10. The material of the electric wiring 40 is not particularly limited, and a material used for a known electric wiring can be employed. As a material of the electric wiring 40, a metal lead wire covered with an insulating material and the like can be mentioned.

Next, a method of using the reactive gas application apparatus 100 will be described. First, the plasma generating gas is supplied to the instrument 10 through the power supply unit 20.

The plasma generating gas supplied to the instrument 10 is introduced from the rear end of the tubular dielectric 3 into the hollow portion of the tubular dielectric 3.

Then, electricity is supplied from the power supply unit 20 to the instrument 10 to apply voltage between the inner electrode 4 and the outer electrode 5. The plasma generating gas introduced into the hollow portion of the tubular dielectric 3 is ionized at a position where the inner electrode 4 and the outer electrode 5 face each other, and turned into plasma.

In this process, electric field concentration occurs on the outer peripheral surface of the inner electrode 4. Since the thread is formed on the inner electrode 4 in the present embodiment, the electric field concentration is caused to dispersedly occur at multiple points on the crest of the thread.

As a result, local overheating of the inner electrode 4 to which the voltage is applied can be prevented, and a low-temperature plasma can easily and stably be generated with a low voltage.

In addition, the reactive gas application apparatus 100 of the present embodiment can stably generate plasma. Therefore, the reactive gas application apparatus 100 can increase the density of the reactive species in the reactive gas to be applied, thereby further promoting cleaning, activation, and healing of the target surface.

In the present embodiment, the inner electrode 4 and the outer electrode 5 face each other in a direction orthogonal to the flowing direction of the plasma generating gas. Plasma generated at a position where the outer peripheral surface of the inner electrode 4 and the inner peripheral surface of the outer electrode 5 face each other is allowed to pass through the gas flow path 6, the first reactive gas flow path 7, and the second reactive gas flow path 8 in this order. In this process, the plasma flows while changing the gas composition, and becomes a reactive gas containing reactive species such as radicals.

The generated reactive gas is released from the outlet 1a and activates a part of the gas in the vicinity of the outlet 1a into reactive species. The reactive gas containing these reactive species is blown out against a target object.

Examples of the target object include cells, living tissues, and whole bodies of organisms.

Examples of the living tissue include various organs such as internal organs, epithelial tissues covering the body surface and the inner surfaces of the body cavity, periodontal tissues such as gums, alveolar bone, periodontal ligament and cementum, teeth, bones and the like.

The whole bodies of organisms may be any of mammals such as humans, dogs, cats, pigs and the like; birds; fish and the like.

Examples of the plasma generating gas include noble gases such as helium, neon, argon and krypton; nitrogen; and the like. With respect to these gases, a single type thereof may be used individually or two or more types thereof may be used in combination.

The plasma generating gas preferably contains nitrogen gas as a main component. Here, the nitrogen gas being contained as a main component means that the volume of the nitrogen gas contained in the plasma generating gas is more than 50 % by volume. Specifically, the volume of the nitrogen gas contained in the plasma generating gas is preferably more than 50 % by volume, more preferably 70 % by volume or more, still more preferably 80 to 100 % by volume, and particularly preferably 90 to 100 % by volume. The gas component other than nitrogen in the plasma generating gas is not particularly limited, and examples thereof include oxygen and a noble gas. By using nitrogen as the main component, the cleaning, activation or healing of the target object can be further promoted. In addition, by using nitrogen as a main component, the oxygen content of the plasma generating gas can be reduced and the ozone content of the reactive gas can be reduced. When the reactive gas application device 10 is used for treatment in the oral cavity, it is preferable to reduce the ozone content of the reactive gas.

With the conventional plasma generating unit, it is difficult to generate plasma with a plasma generating gas containing nitrogen. In the present embodiment, by the use of the inner electrode provided with a spiral ridge on the outer peripheral surface is used, plasma can be easily generated.

When the reactive gas application apparatus 100 is an apparatus for an intraoral treatment, the plasma generating gas to be introduced into the tubular dielectric 3 preferably has an oxygen concentration of 1 % by volume or less. When the oxygen concentration is not more than the upper limit value, generation of ozone can be further suppressed.

The flow rate of the plasma generating gas introduced into the tubular dielectric 3 is preferably 1 to 10 L/min.

When the flow rate of the plasma generation gas introduced into the tubular dielectric 3 is not less than the above lower limit value, it becomes easy to suppress the temperature rise of a target surface of the target object. When the flow rate is not more than the above upper limit value, the cleaning, activation or healing of the target object can be further promoted with ease.

The alternating voltage applied between the inner electrode 4 and the outer electrode 5 is preferably 5 kVpp or more and 20 kVpp or less. Here, the unit "Vpp (Volt peak to peak)" representing the alternating voltage means a potential difference between the highest value and the lowest value of the alternating voltage waveform.

When the applied alternating voltage is not more than the above upper limit value, the temperature of the generated plasma can be kept low. When the applied alternating voltage is not less than the above lower limit value, plasma can be generated more efficiently.

The frequency of the alternating voltage applied between the inner electrode 4 and the outer electrode 5 is preferably 0.5 kHz or more and less than 20 kHz, more preferably 1 kHz or more and less than 15 kHz, even more preferably 2 kHz or more and less than 10 kHz, particularly preferably 3 kHz or more and less than 9 kHz, and most preferably 4 kHz or more and less than 8 kHz.

With the frequency of the alternating voltage set to less than the above upper limit value, the temperature of the generated plasma can be suppressed low. With the frequency of the alternating voltage set to equal or exceed the above lower limit value, plasma can be generated more efficiently.

The temperature of the reactive gas blown out from the outlet 1a of the nozzle 1 is preferably 50 °C or less, more preferably 45 °C or less, and even more preferably 40 °C or less.

When the temperature of the reactive gas blown out from the outlet 1a of the nozzle 1 is not more than the upper limit value, the temperature of the target surface can be easily adjusted to 40 °C or less. By keeping the temperature of the target surface at 40 °C or less, stimulus to the target surface can be reduced even when the target surface is an affected part.

The lower limit value of the temperature of the reactive gas blown out from the outlet 1a of the nozzle 1 is not particularly limited, and is, for example, 10 °C or more.

The temperature of the reactive gas is a temperature value of the reactive gas at the outlet 1a measured by a thermocouple.

The distance (application distance) from the outlet 1a to the target surface is preferably, for example, 0.01 to 10 mm. When the application distance is not less than the above lower limit value, the temperature of the target surface can be lowered, and the stimulus to the target surface can be further reduced. When the application distance is not more than the above upper limit value, the effect of healing and the like can be further enhanced.

The temperature of the reactive gas at a target surface positioned at a distance of 1 mm or more and 10 mm or less from the outlet 1a is preferably 40 °C or less. By setting the temperature of the reactive gas at a target surface to 40 °C or less, stimulus to the target surface can be reduced. The lower limit value of the temperature of the reactive gas at a target surface is not particularly limited, and is, for example, 10 °C or more.

The temperature of the reactive gas at the target surface is adjusted by controlling the alternating voltage applied between the inner electrode 4 and the outer electrode 5, the amount of the blown out reactive gas, the distance from the tip end p of the inner electrode 4 to the outlet 1a, and the like, some or all of which are controlled in combination.

Examples of the reactive species (radicals etc.) contained in the reactive gas include hydroxyl radicals, singlet oxygen, ozone, hydrogen peroxide, superoxide anion radicals, nitric oxide, nitrogen dioxide, peroxynitrite, dinitrogen trioxide and the like. The type of the reactive species contained in the reactive gas can be controlled by, for example, the type of the plasma generating gas.

The hydroxyl radical concentration of the reactive gas (radical concentration) is preferably 0.1 to 300 $\mu$mol/L. When the radical concentration is not less than the lower limit value, the promotion of cleaning, activation or healing of a target object selected from a cell, a living tissue and a whole body of an organism is facilitated. When the radical concentration is not more than the upper limit value, stimulus to the target surface can be reduced.

The radical concentration can be measured, for example, by the following method.

A reactive gas is applied to 0.2 mL of a 0.2 mol/L solution of DMPO (5,5-dimethyl-1-pyrroline-N-oxide) for 30 seconds. Here, the distance from the outlet to a liquid surface of the solution is set to 5.0 mm. With respect to the solution to which the reactive gas has been applied, a hydroxyl radical concentration is measured by electron spin resonance (ESR) method.

The singlet oxygen concentration of the reactive gas is preferably 0.1 to 300 $\mu$mol/L. When the singlet oxygen concentration is not less than the lower limit value, the promotion of cleaning, activation or healing of a target object selected from a cell, a living tissue and a whole body of an organism is facilitated. When the singlet oxygen concentration is not more than the upper limit value, stimulus to the target surface can be reduced.

The singlet oxygen concentration can be measured, for example, by the following method.

A reactive gas is applied to 0.4 mL of a 0.1 mol/L solution of TPC (2,2,5,5-tetramethyl-3-pyrroline-3-carboxamide) for 30 seconds. Here, the distance from the outlet to a liquid surface of the solution is set to 5.0 mm. With respect to the solution to which the reactive gas has been applied, a singlet oxygen concentration is measured by electron spin resonance (ESR) method.

The flow rate of the reactive gas blown out from the outlet 1a is preferably 1 to 10 L/min.

When the flow rate of the reactive gas blown out from the outlet 1 a is not less than the above lower limit value, the effect of the reactive gas acting on the target surface can be sufficiently enhanced. When the flow rate of the reactive gas blown out from the outlet 1a is less than the above upper limit value, excessive increase in the temperature of the reactive gas at the target surface can be prevented. In addition, when the target surface is wet, rapid drying of the target surface can be prevented. Furthermore, when the target surface is an affected part of a patient, stimulus inflicted on the patient can be further suppressed.

In the reactive gas application apparatus 100, the flow rate of the reactive gas blown out from the outlet 1a is adjusted by the supply amount of the plasma generating gas to the tubular dielectric 3.

The reactive gas generated by the reactive gas application apparatus 100 has an effect of promoting healing of trauma and other abnormalities. The application of the reactive gas to a cell, a living tissue or a whole body of an organism can promote cleaning, activation or healing of the target part to which the reactive gas is applied.

Examples of the living tissue include various organs such as internal organs, epithelial tissues covering the body surface and the inner surfaces of the body cavity, periodontal tissues such as gums, alveolar bone, periodontal ligament and cementum, teeth, bones and the like.

Examples of diseases and symptoms that can be treated by application of the reactive gas include diseases in the oral cavity such as gingivitis and periodontal disease, skin wounds and the like.

For applying a reactive gas for the purpose of promoting healing of the trauma and other abnormalities, there is no particular limitation with regard to the interval, repetition number and duration of the application. For example, when a reactive gas is applied to an affected part at a dose of 1 to 5.0 L/min, the application conditions preferred for promoting healing are as follows: 1 to 5 times per day, 10 seconds to 10 minutes for each repetition, and 1 to 30 days as total duration of treatment.

As described above, since the reactive gas application apparatus of the present embodiment includes the inner electrode of a specific configuration, a low-temperature plasma can be generated more stably and the reactive gas generated by the plasma can be applied to the affected part or the like. Therefore, the effect of the plasma can be further enhanced.

The reactive gas application apparatus of the present embodiment applies the reactive gas to a target object. The applied reactive gas can promote tissue repair without damaging the targeted tissue.

The reactive gas application apparatus of the present invention is useful as a medical therapeutic apparatus, particularly useful as an oral cavity treatment apparatus or a dental treatment apparatus.

Further, the reactive gas application apparatus of the present invention is also suitable as an animal treatment apparatus.

Examples

Hereinbelow, the present invention will be described with reference to Examples which, however, should not be construed as limiting the present invention.

(Evaluation Method)

<Would Healing Effect>

A reactive gas was applied to a porcine wound using the reactive gas application apparatus of each of the Examples. The wound before reactive gas application (initial stage) and the wound to which the reactive gas had been applied for 14 days (day 14) were classified according to the following clinical symptom scores, and the score improvement rate was determined by the following formula (1).

$$\text{Score improvement rate } (\%) = ((\text{clinical symptom score (initial stage)} - \text{clinical symptom score (day 14)})/\text{clinical symptom score (initial stage)}) \times 100 \quad (1)$$

[Conditions]

Applied alternating voltage: 16 kVpp.
Frequency of applied voltage: 7.5 kHz.
Reactive gas: nitrogen (purity 99.9%).
Flow rate of reactive gas: 3 L / min.
Reactive gas application time: 60 seconds/time. 1 time/day.
Duration of reactive gas application: 14 days.

[Clinical Symptom Score]

The clinical symptom score was determined by visually checking whether the wounds developed redness, erythema, papule, exudates (including pus) or pustule. The symptoms of the 5 items were scored based on the following criteria by four evaluators and totaled. Each of the points below on the results was an average value of the scores given by the four evaluators.

 0 point: No wound
 1 point: Minor
 2 point: Moderate
 3 point: Severe

<Temperature of Reactive Gas>

In the room with a temperature of 25 °C, a measuring part of thermocouple was positioned 3 mm away from the outlet.
Blowing out of the reactive gas was initiated, and the temperature read 60 seconds after the initiation of the blowing out was taken as the temperature of the blown out reactive gas.

(Example 1)

Substantially the same reactive gas application apparatus as the reactive gas application apparatus 100 was produced, except that the specification was changed as shown below. Using the produced reactive gas application device, the effect of the wound healing was evaluated. The inner electrode had the same configuration as the inner electrode 4 shown in FIG. 2.
As a result, the clinical symptom scores were 13.0 at the initial stage and 1.0 on day 14, and the score improvement rate was 92.3 %.

<Specification>

Tubular dielectric 3: made of glass, inner diameter R = 3 mm.
Inner electrode 4: made of stainless steel, parallel-threaded, single-threaded screw, outer
diameter d = 2 mm, pitch p = 0.4 mm, thread height h = 0.214 mm.
Outer electrode 5: copper plate.
Angle θ: 20 °.

(Comparative Example 1)

Substantially the same reactive gas application apparatus as in Example 1 was produced, except that the inner electrode used had the specification described below. Using the produced reactive gas application device, the effect of the wound healing was evaluated.

<Specification>

Inner electrode 4: made of stainless steel, outer diameter = 2 mm, pitch 0.4 mm, no ridge.
The temperature of the applied gas in Example 1 was 34.9 °C, the radical concentration was 3 $\mu$mol/L, and the singlet oxygen concentration was 3 $\mu$mol/L.
The score improvement rate in Example 1 was 92.3 %, clearly indicating that healing was promoted.
In Example 1 and Comparative Example 1, it was attempted to generate plasma in the reactive gas application apparatus with application of alternating voltages of 7 kVpp and 16 kVpp. In Example 1, plasma was generated at both 7 kVpp and 16 kVpp. In Comparative Example 1, plasma was generated at 16 kVpp, while no plasma was generated at 7 kVpp.
These results revealed that the effect of plasma can be further enhanced by applying the present invention.

Industrial Applicability

The reactive gas application apparatus of the present invention can further enhance the effect of plasma. Therefore, the reactive gas application apparatus of the present invention is suitable as a medical therapeutic apparatus or as an apparatus for treating animals excluding humans.

DESCRIPTION OF THE REFERENCE SIGNS

| | |
|---|---|
| 1 | Nozzle |
| 1a | Outlet |
| 3 | Tubular dielectric |
| 4, 40 | Inner electrode |
| 5 | Outer electrode |
| 12 | Plasma generating unit |
| 42 | Shaft portion |
| 44 | Ridge |
| O1 | Tube axis |

**Claims**

1. A reactive gas application apparatus comprising an instrument,
the instrument comprising:

   a plasma generating unit, and
   an outlet for blowing out a reactive gas activated by plasma,
   the plasma generating unit comprising:

   a tubular dielectric to which a plasma generating gas is introduced;
   an inner electrode provided inside the tubular dielectric and extending in a tube axis direction of the tubular dielectric; and
   an outer electrode provided outside the tubular dielectric and facing the
   inner electrode through the tubular dielectric,

   wherein the inner electrode has a shaft portion extending in the tube axis direction and a helical ridge formed on an outer peripheral surface of the shaft portion.

2. The reactive gas application apparatus according to claim 1, wherein a crest of the helical ridge has an acute angle.

3. The reactive gas application apparatus according to claim 1 or 2, wherein an interval between the crests of neighboring turns of the helical ridge is 0.2 to 3.0 mm as viewed in the tube axis direction.

4. The reactive gas application apparatus according to any one of claims 1 to 3, wherein a height of the helical ridge is 0.1 to 3.0 mm.

5. The reactive gas application apparatus according to any one of claims 1 to 4, wherein a length of a region where the inner electrode faces the outer electrode is 1 to 50 mm.

6. The reactive gas application apparatus according to any one of claims 1 to 5, wherein the plasma generating gas contains nitrogen.

7. The reactive gas application apparatus according to claim 6, wherein the plasma generating gas has a nitrogen content of 80 to 100 % by volume with respect to a total volume of the plasma generating gas.

8. The reactive gas application device according to any one of claims 1 to 7, which is a medical therapeutic apparatus.

9. The reactive gas application apparatus according to any one of claims 1 to 7, which is a therapeutic apparatus for treating animals excluding humans.

10. A method of treating animals excluding humans, comprising providing the reactive gas application apparatus according to any one of claims 1 to 7, and blowing out the reactive gas to a tissue of an animal excluding humans.

FIG. 1

# FIG. 2

EP 3 639 891 A1

# FIG. 3

<u>10</u>

5  2b

12

3

6

4

O1

# FIG. 4

40

44  46  45

42

p2

## INTERNATIONAL SEARCH REPORT

| International application No. |
|---|
| PCT/JP2018/022868 |

| A. CLASSIFICATION OF SUBJECT MATTER |
|---|
| Int.Cl. A61N1/44(2006.01)i, A61C19/06(2006.01)i |
| According to International Patent Classification (IPC) or to both national classification and IPC |

| B. FIELDS SEARCHED |
|---|
| Minimum documentation searched (classification system followed by classification symbols) <br> Int.Cl. A61N1/44, A61C19/06, H05H1/24, H05H1/26 |

| Documentation searched other than minimum documentation to the extent that such documents are included in the fields searched | |
|---|---|
| Published examined utility model applications of Japan | 1922–1996 |
| Published unexamined utility model applications of Japan | 1971–2018 |
| Registered utility model specifications of Japan | 1996–2018 |
| Published registered utility model applications of Japan | 1994–2018 |

Electronic data base consulted during the international search (name of data base and, where practicable, search terms used)

| C. DOCUMENTS CONSIDERED TO BE RELEVANT | | |
|---|---|---|
| Category* | Citation of document, with indication, where appropriate, of the relevant passages | Relevant to claim No. |
| X <br> Y | JP 2012-528452 A (COLORADO STATE UNIVERSITY RESEARCH FOUNDATION) 12 November 2012, paragraphs [0047]-[0050], fig. 1, 7 & US 2011/0101862 A1, paragraphs [0095]-[0098], fig. 1, 7 & WO 2010/138102 A1 & EP 2297377 A1 & AU 2009347177 A & CA 2763870 A | 1 <br> 2-7 |

☒ Further documents are listed in the continuation of Box C.  ☐ See patent family annex.

| * Special categories of cited documents: <br> "A" document defining the general state of the art which is not considered to be of particular relevance <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |
|---|---|

| Date of the actual completion of the international search <br> 11 September 2018 (11.09.2018) | Date of mailing of the international search report <br> 18 September 2018 (18.09.2018) |
|---|---|
| Name and mailing address of the ISA/ <br> Japan Patent Office <br> 3-4-3, Kasumigaseki, Chiyoda-ku, <br> Tokyo 100-8915, Japan | Authorized officer <br><br> Telephone No. |

Form PCT/ISA/210 (second sheet) (January 2015)

**INTERNATIONAL SEARCH REPORT**

International application No.

PCT/JP2018/022868

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2004-342331 A (SEKISUI CHEMICAL CO., LTD.) 12 December 2004, paragraphs [0075], [0080]-[0081], [0093], fig. 2(c), 3(c) (Family: none) | 1-10 |
| Y | JP 2017-50267 A (SEKISUI CHEMICAL CO., LTD.) 09 March 2017, paragraphs [0012]-[0013], [0020], [0040]-[0041], [0047]-[0063], fig. 1-2 (Family: none) | 1-10 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017119153 A
- JP 5441066 B